# EUROPEAN PATENT SPECIFICATION

(11) **EP 3 777 941 B1**
(45) Date of publication and mention of the grant of the patent: **06.09.2023**
(21) Application number: 19776340.2
(22) Date of filing: 12.03.2019
(51) Int. Cl.: A61M 16/04, A61M 29/00

(54) **ENDOTRACHEAL DILATION AND VENTILATION DEVICE**
ENDOTRACHEALE DILATATIONS- UND ATEMVORRICHTUNG
DISPOSITIF DE DILATATION ET DE VENTILATION ENDOTRACHÉAL

(30) Priority: 27.03.2018 ES 201830301
(43) Date of publication of application: 17.02.2021
(73) Proprietor: Gutierrez Gil, Jorge, 46183 L'Eliana (ES); Fundación para la Investigación del Hospital Universitario y Politécnico la Fe de la comunidad Valenciana, 46026 Valencia (ES)
(72) Inventor: GUTIERREZ SAN ROMA, Carlos, 46183 L'Eliana (ES); GUTIERREZ GIL, Jorge, 46183 L'Eliana (ES)
(74) Representative: Isern Patentes y Marcas S.L.
(86) International application number: PCT/ES2019/070164
(87) International publication number: WO 2019/185956

(56) References cited:
- EP-A1- 1 647 233
- EP-A1- 3 135 332
- WO-A1-2010/078148
- WO-A1-2014/163846
- WO-A2-2008/089424
- ES-T3- 2 279 934
- MX-A- 2011 007 038
- MX-A- 2014 001 145
- US-A- 3 880 168
- US-A1- 2014 046 357
- US-A1- 2014 277 058

## Description

### OBJECT OF THE INVENTION

The object of the present application for invention is to register an endotracheal dilation and ventilation device, which incorporates significant innovations and advantages over the techniques used to date.

More specifically, the invention proposes the development of an endotracheal dilation and ventilation device, which due to the particular arrangement thereof, enables a very advantageous use against a tracheal obstruction in a patient, either through the main (oral) route or through an artificial route such as a tracheostomy.

### BACKGROUND OF THE INVENTION

In the known state of the art, there are various documents that are linked in some way, although with substantial differences, to the proposed invention.

For example, document ES2779934T3 relates to an effective pulsion dilator for digestive use, mainly oesophageal, although it refers to the fact that it can be incorporated into a bronchoscope, but it is not suitable for the airway, as it does not enable dilation and ventilation, and it is not designed for being inserted into a narrow airway, and it is not progressive either. It is assumed that it could dilate bronchi, but the act of dilation would clearly affect the patient's ventilation, since the device would occupy the lumen of the trachea, making normal ventilation impossible.

Other provisions known in the state of the art, such as that disclosed by document US2014277058, have generalised the use of pneumatic dilators. A balloon with controlled pressure inflation causes a narrow airway to dilate. However, this manoeuvre leads to apnoea, and therefore can be improved, as it is detrimental to the correct oxygenation of the patient.

In this sense, the arrangement referred to by document MX2014001145A1 uses a dilation inflation balloon around a ventilatory tube, managing to dilate and ventilate the patient at the same time. This arrangement has proven to be effective, but it can be improved since the specific channel to inflate the balloon occupies ventilatory lumen in the device and would hinder correct ventilation in small internal diameters like some serious stenoses. Therefore, this arrangement could be useful, but it does not have the necessary conicity to be applied in certain serious situations that require immediate action, such as severe subglottic stenosis in a newborn, although this need could be complementary to the present invention, such that both devices could act together.

Additionally, this previous disposition would not enable emergency intubation and ventilation in serious situations either, and it is much less intuitive than the proposed invention, as well as substantially more expensive, due to several more complex elements that it incorporates. Therefore, it would not solve the problem of being used in emergency situations and/or in medical facilities with precarious conditions, wherein the device of the proposed invention could be used.

Document EP3135332A1 corresponds to a tracheal catheter to infuse gases once the patient has been intubated with a classic tube. It is a very soft and fine catheter that has nothing to do with the present invention, although it has a slightly conical tip to facilitate insertion into the tracheal tube. It cannot dilate, nor can it intubate, nor can it contain any optics.

Said same document EP3135332A1 and document US3880168A have another similarity with the proposed invention that is only apparent in the conical shape of the design, and may be useful to reach the bronchi in classic intubation and in large airways. However, it is not possible to dilate with these devices, for example, severe stenosis in a newborn.

The present invention contributes to solving the present problem, since it enables use thereof in a very advantageous way against a tracheal obstruction in a patient, either through the main (oral) route or through an artificial route such as a tracheostomy.

### DESCRIPTION OF THE INVENTION

The present invention has been developed in order to provide an endotracheal dilation and ventilation device, which is essentially characterised in that it comprises a tube formed from a first section having a constant internal diameter and a second section having a progressive internal diameter, the second section being in continuity with the first section, and the second section having an internal diameter that decreases after contact with the first section. The free end of the tube that corresponds to the first section is open and able to incorporate a breathing means, and the other opposite free end of the tube that corresponds to the second section is also open and has an outer geometry in the form of a tip with rounded edges.

Preferably, in the endotracheal dilation and ventilation device, the free end of the tube that corresponds to the first section further incorporates an endoscopy means that is able to travel inside the tube through both the first section and the second section and to pass through the opening of the free end of the tube that corresponds to the second section.

Additionally, in the endotracheal dilation and ventilation device, the outer surface of the tube has notches or recesses in the entire or partial longitudinal extension of the tube.

Alternatively, in the endotracheal dilation and ventilation device, the tube is made entirely or partially of biocompatible plastic material, silicone or the like.

According to the invention, the endotracheal dilation and ventilation device, the region of the tube wherein the opening corresponding to the second section is located has less rigid properties than the rest of the same tube.

Alternatively, in the endotracheal dilation and ventilation device, the region of the tube wherein the opening is located has lateral openings or holes capable of improving ventilation.

Alternatively, in the endotracheal dilation and ventilation device, the tube has a straight geometry.

Alternatively, in the endotracheal dilation and ventilation device, the tube has a curved geometry.

Preferably, in the endotracheal dilation and ventilation device, the free end of the tube that corresponds to the first section has a notch therein that is able to adapt to a rigid or flexible optical lens that serves as a guide for the correct insertion of the endoscopic means.

Additionally, the endotracheal dilation and ventilation device incorporates an element with radiopaque properties.

Preferably, in the endotracheal dilation and ventilation device, the element with radiopaque properties comprises a metal part in the form of a ring and embedded in the tube.

Preferably, in the endotracheal dilation and ventilation device, the metal part in the form of a ring is arranged in the region of the tube wherein the first section makes contact with the second section.

Alternatively, in the endotracheal dilation and ventilation device, the endoscopy means is linked by means of a cable or wirelessly to a device such as a tablet or smartphone that incorporates an application enabled for this purpose.

The present invention provides a very advantageous use against a tracheal obstruction in a patient, either through the main (oral) route or through an artificial route such as a tracheostomy.

Other characteristics and advantages of the endotracheal dilation and ventilation device will be apparent from the description of a preferred, but not exclusive, embodiment, which is illustrated by way of non-limiting example in the accompanying drawings, in which:

### BRIEF DESCRIPTION OF THE DRAWINGS

Figure 1 is a schematic view of a preferred embodiment of the endotracheal dilation and ventilation device of the present invention.
Figures 2 and 3 are schematic views of a preferred embodiment of the endotracheal dilation and ventilation device of the present invention with an incorporated endoscopic and cylindrical means.
Figure 4 is a schematic view of a preferred embodiment of the endotracheal dilation and ventilation device of the present invention having a curved geometry.
Figure 5 is a schematic view of a preferred embodiment of the endotracheal dilation and ventilation device of the present invention also having a curved geometry and having an incorporated endoscopic means.

### DESCRIPTION OF A PREFERRED EMBODIMENT

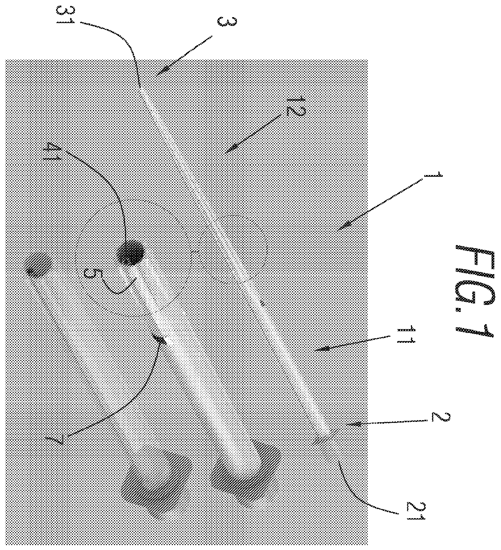

As shown schematically in Figure 1, the endotracheal dilation and ventilation device comprises a tube 1 formed from a first section 11 having a constant internal diameter and a second section 12 having a progressive internal diameter, the second section 12 being in continuity with the first section 11.

The second section 12 of the tube 1 has an internal diameter that decreases after contact with the first section 11 of the same tube 1.

The free end 2 of the tube 1 that corresponds to the first section 11 is open with an opening 21 and able to incorporate a breathing means, either manual or mechanical.

The other opposite free end 3 of the same tube 1 that corresponds to the second section 12 is also open with an opening 31 and has an outer geometry in the form of a tip with rounded edges.

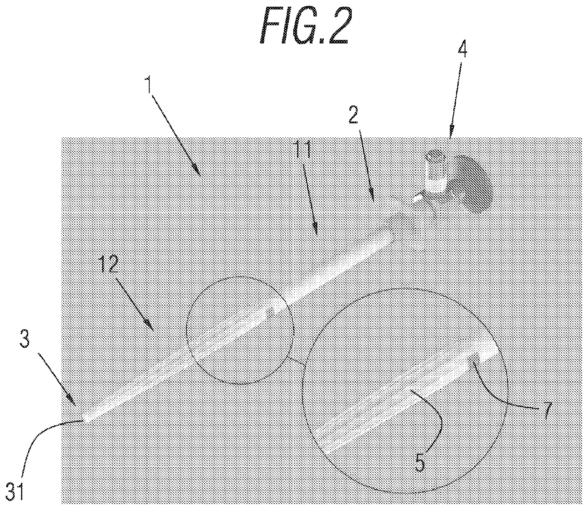

The free end 2 of the tube 1 that corresponds to the first section 11 further incorporates a cylindrical endoscopy means 4 that is able to travel inside the tube 1 through both the first section 11 and the second section 12 and to pass through the opening 31 of the free end 3 of the tube 1 that corresponds to the second section 12, as shown in Figure 2.

This internal cylindrical endoscopy means 4 can be useful to insert a thin guidewire 41 as shown in the enlarged detail of Figure 1, to facilitate the insertion of the endotracheal dilation and ventilation device of the invention, to vacuum secretions or to use a "jet" ventilation system which consists of the administration of oxygen at controlled pressure to facilitate ventilation in situations of airway stricture.

The outer surface of the tube 1 has notches 5 or recesses in the entire or partial longitudinal extension of the tube 1, as shown in the enlarged details of Figures 1 and 2.

Tube 1 is made of biocompatible plastic material, silicone or the like. In addition, the region of the tube 1 wherein the opening 31 is located has less rigid properties than the rest of the same tube 1, or with semi-rigid properties, even also made of silicone or the like, in order to avoid and prevent injuries at the time of inserting it into the patient.

The endotracheal dilation and ventilation device of the invention enables the strictures of the patient's airway to be dilated by pulsion and advance thereof. It manages to break the granulation tissue in the mucosa of the stricture and establish a new internal diameter. At the same time and once the optics have been removed from the endoscopic means 4, it enables the patient to be correctly ventilated since the design in the proximal portion thereof adapts to the breathing means, be it the breathing means of the anaesthetic tower or a manual ventilator, for example.

The length, diameter and internal diameters of tube 1 are variable to adapt to the different ages and characteristics of the patient.

The external notches 5 or recesses facilitate the breaking of the narrow ring of the patient.

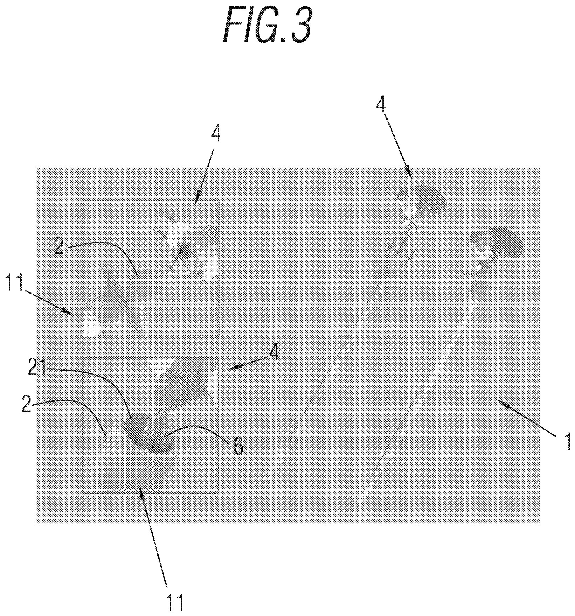

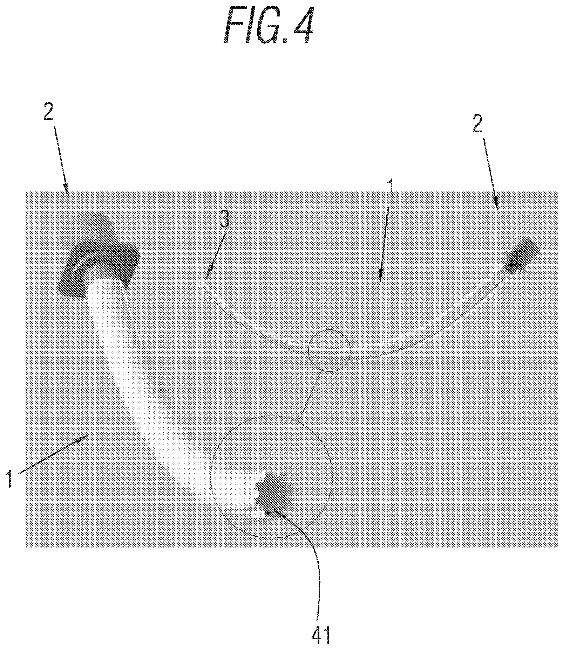

The end 2 of the tube 1 that corresponds to the first section 11 has a notch 6 therein that is able to adapt to a rigid optical lens that serves as a guide for the correct insertion of the endoscopic means 4 adapted for this purpose, as shown in the enlarged detail of Figure 3. The guidewire 41 can also be used in this sense, as shown in Figures 1 and 4.

The tube 1 can have a straight geometry, as shown in Figures 1, 2 and 3, thus enabling the initial insertion of a rigid endoscopic means 4, especially useful when the patient's neck is extended and, consequently, the airway is straight.

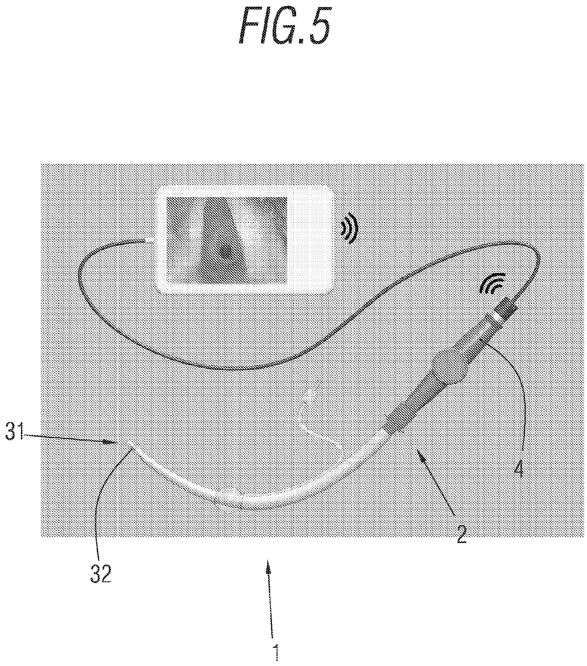

The tube 1 can also have a curved geometry, as shown in Figures 4 and 5, thus allowing for a flexible endoscopic means 4, useful when the patient's neck is flexed.

Figure 5 shows how the region of the tube 1 wherein the opening 31 is located has lateral openings or holes 32 and a balloon to thus improve ventilation. It also shows how the endoscopic means 4 uses a wired screen or a wireless application APP with a table or smartphone.

When using the endotracheal dilation and ventilation device of the invention, once the stricture has been diagnosed by means of rigid or flexible endoscopy, the tube 1 of the endotracheal dilation and ventilation device of the invention is inserted into the patient's trachea and moves forward through the interior thereof until the stenosis is reached. With direct vision, it moves forward along the stricture, the endoscopic means 4 and the optics thereof are removed, it is connected to the breathing means, and dilation is achieved by pulsion.

The tube 1 of the endotracheal dilation and ventilation device of the invention can keep dilating for the time required since the patient is perfectly ventilated since the start of the procedure.

The endotracheal dilation and ventilation device of the invention is designed for tracheal use both in the subglottic region and in the trachea and main bronchi, modifying the length thereof.

Tubes 1 having smaller internal diameter will be used in newborn infants with subglottic stenoses, and tubes 1 having larger internal diameter are useful for older children and adults.

Other preferred embodiments of the endotracheal dilation and ventilation device of the invention can incorporate an element with radiopaque properties, so that in an X-ray examination of the patient, an actual position of the endotracheal dilation and ventilation device of the invention can be obtained.

Said element with radiopaque properties may comprise a metal part 7 in the form of a ring, which due to the metal nature thereof has radiopaque properties, and which is embedded in the tube 1 and which covers a good portion of the circular perimeter of the tube 1, and which is arranged in the region of the tube 1 wherein the first section 11 makes contact with the second section 12.

The endotracheal dilation and ventilation device of the invention could further be used to dilate pharyngeal, tracheostomas and oesophageal stenoses.

More specifically, subglottic stenosis is known as one of the most problematic incidents in neonatal and paediatric intensive treatment areas.

These patients often require long periods of ventilator-assisted ventilation, which injures the subglottic region thereof. Ventilatory failures that require urgent re-intubation may occur, in this case with an internal diameter that is smaller than the previous one of the newborn, due to the presence of inflammatory tissue caused by the tube.

There are also situations that require the use of a very small internal diameter to secure the airway and prevent an emergency tracheostomy. In these situations, the endotracheal dilation and ventilation device of the proposed invention can be very useful, on the one hand, by ensuring ventilation, and on the other hand, by dilating the granulation tissue and avoiding an emergency tracheostomy.

In the known state of the art, the trachea is generally dilated with pneumatic dilators, but it is necessary to have low-cost devices that can replace them in small internal diameters, or that can complement pneumatic dilation, as is the case with the endotracheal dilation and ventilation device of the proposed invention.

Another point of interest of the endotracheal dilation and ventilation device of the present invention is the possibility of use thereof in the management of difficult airways (DA), which is defined as a clinical situation in which a trained professional experiences difficulty with face mask ventilation, with tracheal intubation, or both. Even these difficulties can lead to the inability to ventilate a patient and cause the death thereof.

It is known that an accidental rupture of the trachea (0.4 % of accidents) can occur in obese patients, pregnant patients and those with craniofacial malformations. 34 % of anaesthetic lawsuits are associated with DA. This frequency is important since a proportion of 1/50,000 patients cannot be intubated, and intubation failures occur in a proportion of 1/2,000 patients with scheduled anaesthesia and 1/200 emergency patients.

The endotracheal dilation and ventilation device of the present invention may be especially useful in DA. Currently, DA is approached with intubation by means of an endoscope, but with the same intubation tubes as in the non-problematic airway. In the state of the art, it is reported that this method is involved in 8.5 % of vocal cord injuries (Anaesthesiology 2007, 107: 585-90), which could be prevented with the use of a tube having a progressive internal diameter with the endoscope at the tip, as referred to in the endotracheal dilation and ventilation device of the proposed invention. The association of progressive internal diameter and rigid or flexible optical elements enables the vocal cords and also the trachea to be properly viewed, which represents an advantage in DA over other devices in the state of the art.

In situations in which an endoscope is not available, such as ambulances and centres in geographic areas without financial means, the use of the endotracheal dilation and ventilation device of the present invention is possible in the working channel mode. The narrow airway is viewed, a thin guidewire with a soft tip is inserted and then the present endotracheal dilation and ventilation device of the proposed invention is placed, capable of solving emergency ventilation with very small internal diameters of useful air lumen and with low-cost equipment.

When a reduced internal diameter is used and it is not sufficient to correctly ventilate the patient, since it has been dilated, it is possible to use an immediately larger size, keeping the guide until proper ventilation is achieved.

The endotracheal dilation and ventilation device of the proposed invention provides three important improvements to the known state of the art.

In the first place, both in surgery and other medical applications, as well as in emergency situations in which advanced means are not available, the endotracheal dilation and ventilation device of the present invention proposes an advanced technical solution, since communication of the airway (tracheal dilation) is obtained and instant ventilation is provided to the patient.

Secondly, the airway can be secured either by an endoscopic-type optical element (flexible or rigid) or by using an internal working channel (very thin tube) that can provide guidance for the endotracheal dilation and ventilation device of the present invention through a very narrow airway.

Thirdly, the possibility of using a flexible endoscopy means in the embodiment of the endotracheal dilation and ventilation device of the present invention having curved geometry. The endotracheal dilation and ventilation device of the present invention is able to adapt, for difficult intubation, in the event that patients do not present an extended neck and therefore an intubation with elements of rigid or straight geometry would be less preferable.

The incorporation of optical elements with wireless image capture, in addition to reducing the price, favours autonomy and therefore being able to easily move the device to the patient, which is very important in emergency situations.

Both the rectilinear version and the curved geometry version of the endotracheal dilation and ventilation device of the present invention coincide in the technical application of solving a joint problem, as well as, after prior assessment by the physician in charge of the intervention, being able to use one or the other version, depending on the patient's conditions as well as their own experience.

In addition to all that has been explained, due to the intuitiveness and practicality of the endotracheal dilation and ventilation device of the proposed invention, with a brief training, any physician or healthcare personnel will be able to use the endotracheal dilation and ventilation device of the invention, especially when they are treating patients in emergency situations, with vital risk in outdoor interventions, in ambulances, in health centres wherein there is no operating theatre or any intervention rooms, as well as expert personnel in anaesthesia and resuscitation, always providing assurance regarding the air permeability of a patient.

Due to the technical simplicity of the endotracheal dilation and ventilation device of the proposed invention, it can be part of a therapeutic arsenal, as well as part of the emergency kit of any hospital centre, or part of difficult airways equipment in any hospital facility, since, for economic purposes, the impact is much lower than with many other devices of the state of the art.

The endotracheal dilation and ventilation device of the invention additionally envisages the incorporation of progressive internal diameters in order to provide progressive intubations depending on the section of each one of them, to be able to start dilating the airway with a first internal diameter of reduced dimensions, and progressively use devices with sections that multiply the previous one and dilate the airway lumen, and therefore progressively improve the ventilation of the patient.

Quantifying the above, and by way of an illustrative and non-limiting example, a series of internal diameters could be available for the second section 12 having an internal diameter that decreases after contact with the first section 11, as indicated below:
- A first lumen of 2.5 mm to 4 mm of internal diameter.
- A first lumen of 3.5 mm to 8 mm of internal diameter.
- A first lumen of 7.5 mm to 12 mm of internal diameter.

The endotracheal dilation and ventilation device of the proposed invention may be useful for use even in the veterinarian sector.

The details, shapes, dimensions and other accessory elements, as well as the materials used in the manufacture of the present endotracheal dilation and ventilation device may be suitably substituted with others that are technically equivalent and do not depart from the essential features of the invention or from the scope defined by the claims included below.

## Claims

1. An endotracheal dilation and ventilation device, wherein that it comprises a tube (1) formed from a first section (11) having a constant internal diameter and a second section (12) having a progressive internal diameter, the second section (12) being in continuity with the first section (11), and the second section (12) having an internal diameter that decreases after contact with the first section (11), and the free end (2) of the tube (1) that corresponds to the first section (11) being open with an opening (21) and able to incorporate a breathing means, and the other opposite free end (3) of the tube (1) that corresponds to the second section (12) is also open with an opening (31) and has an outer geometry in the form of a tip with rounded edges; and the region of the tube (1) wherein the opening (31) at the end (3) of the tube (1) that corresponds to the second section (12) is located has less rigid properties than the rest of the same tube (1).

2. The endotracheal dilation and ventilation device according to claim 1, **characterised in that** the free end (2) of the tube (1) that corresponds to the first section (11) further incorporates an endoscopy means (4) that is able to travel inside the tube (1) through both the first section (11) and the second section (12) and to pass through the opening (31) of the free end (3) of the tube (1) that corresponds to the second section (12).

3. The endotracheal dilation and ventilation device according to claim 1, **characterised in that** the outer surface of the tube (1) has notches (5) or recesses in the entire or partial longitudinal extension of the tube (1).

4. The endotracheal dilation and ventilation device according to claim 1, **characterised in that** the tube (1) is made entirely or partially of biocompatible plastic material, silicone or the like.

5. The endotracheal dilation and ventilation device according to claim 1, **characterised in that** the region of the tube (1) wherein the opening (31) is located has lateral openings or holes (32) capable of improving ventilation.

6. The endotracheal dilation and ventilation device according to claim 1, **characterised in that** the tube (1) has a straight geometry.

7. The endotracheal dilation and ventilation device according to claim 1, **characterised in that** the tube (1) has a curved geometry.

8. The endotracheal dilation and ventilation device according to claim 2, **characterised in that** the free end (2) of the tube (1) that corresponds to the first section (11) has a notch (6) therein that is able to adapt to a rigid or flexible optical lens that serves as a guide for the correct insertion of the endoscopic means (4).

9. The endotracheal dilation and ventilation device according to any of the preceding claims, **characterised in that** it incorporates an element with radiopaque properties.

10. The endotracheal dilation and ventilation device according to claim 9, **characterised in that** the element with radiopaque properties comprises a metal part (7) in the form of a ring and embedded in the tube (1).

11. The endotracheal dilation and ventilation device according to claim 10, **characterised in that** the metal part (7) in the form of a ring is arranged in the region of the tube (1) wherein the first section (11) makes contact with the second section (12).

12. The endotracheal dilation and ventilation device according to claim 2, **characterised in that** the endoscopy means (4) is linked by means of a cable or wirelessly to a device such as a tablet or smartphone that incorporates an application enabled for this purpose.

## Patentansprüche

1. Endotracheale Dilatations- und Belüftungsvorrichtung, wobei sie ein Rohr (1) umfasst, das aus einem ersten Abschnitt (11), der einen konstanten Innendurchmesser aufweist, und einem zweiten Abschnitt (12), der einen progressiven Innendurchmesser aufweist, ausgebildet ist, wobei der zweite Abschnitt (12) in Kontinuität mit dem ersten Abschnitt (11) steht und der zweite Abschnitt (12) einen Innendurchmesser aufweist, der nach dem Kontakt mit dem ersten Abschnitt (11) abnimmt, und das freie Ende (2) des Rohres (1), das dem ersten Abschnitt (11) entspricht, mit einer Öffnung (21) offen ist und ein Atmungsmittel aufnehmen kann, und das andere, gegenüberliegende freie Ende (3) des Rohres (1), das dem zweiten Abschnitt (12) entspricht, ebenfalls mit einer Öffnung (31) offen ist und eine Außengeometrie in Form einer Spitze mit abgerundeten Kanten aufweist; und der Bereich des Rohres (1), in dem sich die Öffnung (31) an dem Ende (3) des Rohres (1) befindet, die dem zweiten Abschnitt (12) entspricht, weniger steife Eigenschaften aufweist als der Rest desselben Rohres (1).

2. Endotracheale Dilatations- und Belüftungsvorrichtung nach Anspruch 1, **dadurch gekennzeichnet, dass** das freie Ende (2) des Rohres (1), das dem ersten Abschnitt (11) entspricht, ferner ein Endoskopiemittel (4) enthält, das im Inneren des Rohres (1) sowohl durch den ersten Abschnitt (11) als auch durch den zweiten Abschnitt (12) verlaufen und durch die Öffnung (31) des freien Endes (3) des Rohres (1), das dem zweiten Abschnitt (12) entspricht, hindurchgeführt werden kann.

3. Endotracheale Dilatations- und Belüftungsvorrichtung nach Anspruch 1, **dadurch gekennzeichnet, dass** die Außenfläche des Rohres (1) in der gesamten oder teilweisen Längserstreckung des Rohres (1) Einkerbungen (5) oder Aussparungen aufweist.

4. Endotracheale Dilatations- und Belüftungsvorrichtung nach Anspruch 1, **dadurch gekennzeichnet, dass** das Rohr (1) ganz oder teilweise aus biokompatiblem Kunststoff, Silikon oder dergleichen besteht.

5. Endotracheale Dilatations- und Belüftungsvorrichtung nach Anspruch 1, **dadurch gekennzeichnet, dass** der Bereich des Rohres (1), in dem sich die Öffnung (31) befindet, seitliche Öffnungen oder Löcher (32) aufweist, die die Belüftung verbessern können.

6. Endotracheale Dilatations- und Belüftungsvorrichtung nach Anspruch 1, **dadurch gekennzeichnet, dass** das Rohr (1) eine gerade Geometrie aufweist.

7. Endotracheale Dilatations- und Belüftungsvorrichtung nach Anspruch 1, **dadurch gekennzeichnet, dass** das Rohr (1) eine gekrümmte Geometrie aufweist.

8. Endotracheale Dilatations- und Belüftungsvorrichtung nach Anspruch 2, **dadurch gekennzeichnet, dass** das freie Ende (2) des Rohres (1), das dem ersten Abschnitt (11) entspricht, darin eine Einkerbung (6) aufweist, die sich an eine steife oder flexible optische Linse anpassen kann, die als Führung für das korrekte Einführen des endoskopischen Mittels (4) dient.

9. Endotracheale Dilatations- und Belüftungsvorrichtung nach einem der vorhergehenden Ansprüche, **dadurch gekennzeichnet, dass** sie ein Element mit röntgendichten Eigenschaften enthält.

10. Endotracheale Dilatations- und Belüftungsvorrichtung nach Anspruch 9, **dadurch gekennzeichnet, dass** das Element mit röntgendichten Eigenschaften ein Metallteil (7) in Form eines Rings und eingebettet in das Rohr (1) umfasst.

11. Endotracheale Dilatations- und Belüftungsvorrichtung nach Anspruch 10, **dadurch gekennzeichnet, dass** das Metallteil (7) in Form eines Ringes in dem Bereich des Rohres (1) angeordnet ist, in dem der erste Abschnitt (11) mit dem zweiten Abschnitt (12) in Kontakt steht.

12. Endotracheale Dilatations- und Belüftungsvorrichtung nach Anspruch 2, **dadurch gekennzeichnet, dass** das Endoskopiemittel (4) über ein Kabel oder drahtlos mit einer Vorrichtung wie einem Tablet oder Smartphone verbunden ist, die eine für diesen Zweck geeignete Anwendung enthält.

## Revendications

1. Dispositif de dilatation et de ventilation endotrachéale, dans lequel il comprend un tube (1) formé d'un premier tronçon (11) ayant un diamètre interne constant et d'un deuxième tronçon (12) ayant un diamètre interne progressif, le deuxième tronçon (12) étant en continuité avec le premier tronçon (11), et le deuxième tronçon (12) ayant un diamètre interne qui diminue après contact avec le premier tronçon (11), et l'extrémité libre (2) du tube (1) qui correspond au premier tronçon (11) étant ouverte par une ouverture (21) et apte à incorporer un moyen de respiration, et l'autre extrémité libre opposée (3) du tube (1) qui correspond au deuxième tronçon ( 12) est également ouverte par une ouverture (31) et a une géométrie externe sous la forme d'une pointe avec des bords arrondis ; et la région du tube (1) dans laquelle l'ouverture (31) au niveau de l'extrémité (3) du tube (1) qui correspond au deuxième tronçon (12) est située a des propriétés moins rigides que le reste du même tube (1).

2. Dispositif de dilatation et de ventilation endotrachéale selon la revendication 1, **caractérisé en ce que** l'extrémité libre (2) du tube (1) qui correspond au premier tronçon (11) incorpore en outre un moyen d'endoscopie (4) qui est apte à se déplacer à l'intérieur du tube (1) à travers à la fois le premier tronçon (11) et le deuxième tronçon (12) et à traverser l'ouverture (31) de l'extrémité libre (3) du tube (1) correspondant au deuxième tronçon (12).

3. Dispositif de dilatation et de ventilation endotrachéale selon la revendication 1, **caractérisé en ce que** la surface externe du tube (1) a des encoches (5) ou évidements dans tout ou partie de l'extension longitudinale du tube (1).

4. Dispositif de dilatation et de ventilation endotrachéale selon la revendication 1, **caractérisé en ce que** le tube (1) est fait en tout ou partie de matière plastique biocompatible, de silicone ou similaire.

5. Dispositif de dilatation et de ventilation endotrachéale selon la revendication 1, **caractérisé en ce que** la région du tube (1) dans laquelle l'ouverture (31) est située a des ouvertures ou trous latéraux (32) capables d'améliorer la ventilation.

6. Dispositif de dilatation et de ventilation endotrachéale selon la revendication 1, **caractérisé en ce que** le tube (1) a une géométrie rectiligne.

7. Dispositif de dilatation et de ventilation endotrachéale selon la revendication 1, **caractérisé en ce que** le tube (1) a une géométrie courbe.

8. Dispositif de dilatation et de ventilation endotrachéale selon la revendication 2, **caractérisé en ce que** l'extrémité libre (2) du tube (1) qui correspond au premier tronçon (11) a une encoche (6) dans celle-ci qui est apte à s'adapter à une lentille optique rigide ou souple en guise de guide pour l'insertion correcte du moyen endoscopique (4).

9. Dispositif de dilatation et de ventilation endotrachéale selon l'une quelconque des revendications précédentes, **caractérisé en ce qu'**il incorpore un élément avec des propriétés radio-opaques.

10. Dispositif de dilatation et de ventilation endotrachéale selon la revendication 9, **caractérisé en ce que** l'élément avec des propriétés radio-opaques comprend une pièce métallique (7) sous la forme d'un anneau et incorporée dans le tube (1).

11. Dispositif de dilatation et de ventilation endotrachéale selon la revendication 10, **caractérisé en ce que** la pièce métallique (7) sous la forme d'un anneau est agencée dans la zone du tube (1) dans laquelle le premier tronçon (11) entre en contact avec le deuxième tronçon (12).

12. Dispositif de dilatation et de ventilation endotrachéale selon la revendication 2, **caractérisé en ce que** le moyen d'endoscopie (4) est relié au moyen d'un câble ou sans fil à un dispositif tel qu'une tablette ou un smartphone qui incorpore une application activée à cet effet.
